# EUROPEAN PATENT APPLICATION

(11) **EP 1 138 776 A1**
(43) Date of publication of application: **04.10.2001**
(21) Application number: 00106861.8
(22) Date of filing: 30.03.2000
(51) Int. Cl.: C12Q 1/02

(54) **Cell-free reconstitution of autophagocytosis**

(71) Applicant: Max-Planck-Gesellschaft zur Förderung der Wissenschaften e.V., 80539 München (DE)
(72) Inventor: Mayer, Andreas, Dr., 72076 Tübingen (DE)
(74) Representative: Weiss, Wolfgang, Dipl.-Chem. Dr.

(57) **Abstract**

The present invention relates to a method for determining autophagocytosis activity in a cell-free system and test kits suitable for carrying out this method. Particularly, a method for determining, if a test substance is a modulator, e.g. activator or inhibitor of autophagocytosis, is disclosed. Further, the invention relates to a method for determining autophagocytosis activity in a sample, particulary in a human tissue or body fluid sample.

## Description

The present invention relates to a method for determining autophagocytosis activity in a cell-free system and test kits suitable for carrying out this method. Particularly, a method for determining, if a test substance is a modulator, e.g. activator or inhibitor of autophagocytosis, is disclosed. Further, the invention relates to a method for determining autophagocytosis activity in a sample, particulary in a human tissue or body fluid sample.

Eukaryotic cells have developed multiple pathways for the intracellular degradation of proteins. The proteasome system and direct translocation of proteins through the lysosomal membrane account for only part of the protein turnover in eukaryotes (Cuervo and Dice, 1998). A major route of degradation is autophagocytosis, a process of controlled self-digestion of cells. Autophagocytosis is constitutive, but it is strongly enhanced under conditions of stress, starvation, or redifferentiation (Knop et al., 1993; Seglen and Bohley, 1992). A wide range of eukaryotic cells are capable of autophagocytosis, indicating that the process may be a general asset of eukaryotic life.

Current models differentiate two forms of autophagocytosis, macro- and microautophagocytosis. Macroautophagocytosis is characterized by the appearance of a unique type of vesicles. These vesicles (autophagosomes) sequester portions of cytosol. They also can engulf entire compartments like mitochondria or peroxisomes. Autophagosomes are surrounded by two or sometimes several membranes which clearly distinguish them from other cellular vesicles. The outer membrane of autophagosomes can fuse with lysosomes, delivering the inner membrane with its cytosolic contents (the autophagic body) into the lysosomal lumen for degradation (Seglen and Bohley, 1992). Though macroautophagocytosis has been morphologically well defined, the molecular mechanisms of autophagosome formation and fusion with vacuoles/lysosomes are still poorly understood. Also the origin of the autophagosomal membrane is controversial (Yamamoto et al., 1990a,b; Dunn 1990).

Microautophagocytosis is defined as a direct invagination of the lysosomal membrane. It results in lysosomal vesicles pinching off into the lumen of the lysosome yielding autophagic bodies which are finally degraded. Also the microautophagic pathway is capable of delivering not only soluble proteins but entire organelles into lysosomes for degradation (Veenhuis et al., 1983; Yokota, 1993; Tuttle and Dunn, 1993; Tuttle and Dunn, 1995). It is completely unknown which mechanisms drive the profound changes in lysosomal structure during microautophagic invagination and scission of the nascent autophagic body.

Different approaches have been taken to analyze autophagocytosis in various systems. Pharmacological studies have defined agents inducing or inhibiting autophagocytosis in cell culture systems, perfused organs or entire animals (Seglen and Bohley, 1992; Mortimore and Kadowaki, 1994). Considerable efforts were also made to isolate autophagosomes (Stromhaug et al., 1998), a prerequisite for analyzing their protein composition, identifying marker proteins and analyzing their origin.

Pioneering genetic screens in Saccharomyces cerevisiae have revealed more than 15 complementation groups of mutants defective in autophagocytosis, providing a solid basis for the molecular analysis of the process (Tsukada et al., 1993; Thumm et al., 1994). These complementation groups overlap strongly with a set of mutants defective in the cvt (cytoplasm to vacuole targeting) pathway (Harding et al., 1996; Scott et al., 1996). The cvt pathway leads to the selective and rapid import of aminopeptidase I into vacuoles and occurs via vesicular intermediates structurally related to autophagosomes (Baba et al., 1997). Most of the affected genes have now been identified, exploiting the fact that autophagocytosis in yeast is essential for surviving starvation and for sporulation. Their gene encode presumptive regulatory components like a protein kinase (Apg1p/Aut3p; Matsuura et al., 1997; Straub et al., 1997). A ubiquitin-like protein conjugation system was found comprising an activation enzyme (Apg7p), a ligase (Apg10p) and two proteins (Apg12p and Apg5p) which become covalently attached to each other via these enzymes (Mitzushima et al., 1998; Kim et al., 1999; Shintani et al., 1999). A ubiquitin activating enzyme is also involved in the autophagic pathway of mammalian cells (Lenk et al., 1992). Homo-oligomers of Apg16p bind to the Apg5p-Apg12p conjugate (Mitzushima et al., 1999). Aut2p and Aut7p were proposed to bind to microtubules and mediate the delivery of autophagosomes to the vacuole (Lang et al., 1998) This interpretation was challenged, however, by the recent finding that nocodazole treatment - disassembling microtubules-does not block autophagocytosis in yeast (Kirisako et al. 1999). Apg8p is involved in autophagosome formation. It localizes to the membrane of autophagosome precursors in the cytosol as well as to autophagic bodies, providing the first marker protein for autophagosomes (Kirisako et al., 1999). Genetic screens in Pichia pastoris and Hansenula polymorpha have identified another set of mutations affecting peroxisome degradation (Titorenko et al., Yuan et al., 1999; Sakai et al., 1998). Apg7p is necessary for the degradation of peroxisomes, demonstrating that autophagoxytosis of soluble proteins and peroxisomes may employ a common machinery (Yuan et al., 1999; Kim et al., 1999).

The macroautophagic pathway in Saccharomyces cerevisiae has been extensively characterized by light and electron microscopy (Takeshige et al., 1992; Baba et al., 1994 and 1995). The pathway depends on Vam7p and Vam3p, two SNARE proteins which are presumably involved in fusing the outer membrane of autophagosomes with the vacuole (Darsow et al., 1997; Sato et al., 1998). Therefore, macroautophagocytosis seems to involve the conserved fusion enzymes that also mediate membrane fusion at many other steps of vesicular trafficking (Rothman, 1994). We have recently discovered a microautophagic pathway in Saccharomyces (Müller et al., submitted). There, a specialized structure, the autophagic tube, mediates invagination of the vacuolar membrane and scission of vesicles into the lumen. This tube displays a striking lateral heterogeneity of membrane structure. The zone of vesicle formation at the tip of the tube is devoid of visible transmembrane particles, as is the membrane of autophagic bodies (Müller et al., submitted). In line with this, we found small areas with very low content of transmembrane particles on the vacuolar surface. These smooth areas often invaginate and appear to be precursors of autophagic tubes.

In order to study the mechanisms behind these profound changes of membrane structure and organellar morphology biochemically, we have now reconstituted autophagocytosis in vitro. We have developed a system based on isolated yeast vacuoles and devised fast assays permitting light microscopic tracing of the reaction as well as convenient quantitation. We have utilized this system to characterize the requirements of autophagic protein uptake into vacuoles and/or lysosomes.

A first aspect of the present invention is a method for determining, if a test substance is a modulator of autophagocytosis, comprising the steps:
(a) providing a cell-free autophagocytosis test system comprising a detectable substrate which is capable of being subjected to autophagocytosis in said test system under test conditions and
(b) determining the influence of the test substance on the autophagocytosis of said substrate.

The method of the invention allows the determination of activators or inhibitors of autophagocytosis. Autophagocytosis is a major pathway of protein degradation and particularly important under starvation and stress conditions, but also for development and differentiation processes. Further, autophagocytosis is also related to tumor diseases, in particular to the generation of breast cancer. Finally, autophagocytosis is also connected with active forms of cell death, e.g. apoptosis. Thus, the method of the invention allows the identification of substances which are modulators of pathogenic conditions which are associated with autophagocytosis, particularly with aberrant autophagocytosis.

The method according to the invention is an in vitro test system, which allows a simple means of detecting the influence of a variation of chemical compositions on autophagocytosis. Thus, the method is very important for commercial applications.

Preferably, the cell-free autophagocytosis test system of the invention comprises a source of eukaryotic lysosomes, i.e. lysosomal preparations capable of autophagocytosis. More preferably, this source of lysosomes comprises vacuoles from a yeast cell. However, also lysosomal preparations from higher cells, e.g. vertebrate cells, in particular mammlian cells, can be used. Furthermore, the cell-free autophagocytosis test system comprises a cytosol extract from a eukaryotic cell. This cytosol extract is preferably derived from a yeast cell, in particular from a yeast cell, which was cultivated under starvation conditions. Also here suitable cytosol extracts from higher cells, e.g. from vertebrate or mammalian cells can be used. A further important component of the test system is an energy regeneration system, which provides the energy equivalents necessary for autophagocytosis. A preferred energy regeneration system is an ATP regenerating system, which in a particularly preferred embodiment comprises ATP, creatine phosphate and creatine kinase.

A further important component of the method according to the invention is a detectable substrate which is capable of being subjected to autophagocytosis in the presence of the test system and under the test conditions. Generally, any substrate is suitable which does not spontaneously pass a lysosomal membrane and which may be detected with chemical, biological or physical methods. The substrate may contain labelling groups, preferably fluorescence labelling groups, however, all other forms of detectable labelling groups or compounds are suitable as substrates such as reporter enzymes, radiolabeled substrates or coloured substrates. Preferred examples for substrates are fluorescence-labeled compounds, e.g. carrier molecules having coupled thereto fluorescence groups such as fluoresceine, e.g fluoresceine-labeled dextrane, and reporter enzymes such as firefly luciferase or GFP.

The detection of autophagocytosis activity comprises a selective and optionally quantitative detection of autophagocytosed substrate and/or non-autophagocytosed substrate. If a polypeptide is used as substrate, the detection can e.g. comprise an inactivation, e.g. by degrading or chemical inactivation by enzyme inhibitors of non-autophagocytosed substrate and subsequently determining the autophagocytosed substrate. For example, after the authophagocytosis activity has been terminated, the test system can be incubated with proteinases, e.g. proteinase K, wherein non-autophagocytosed proteins are degraded. After the protease activity has been terminated, and after lysis of the lysosomal membrane by detergents, the autophagocytosed protein can be determined.

Substances determined as autophagocytosis modulators by the method according to the invention or substances derived from further tests can be provided as pharmacological agents for modulating autophagocytosis activity. The present invention also comprises the pharmaceutical application of substances identified by the method as described above. Examples for already identified autophagocytosis modulators are F48/80 and GTP-yS.

The term "test substance" in accordance with the present invention refers to any substance for which a possible modulating activity on autophagocytosis may be tested. The term comprises a single substance as well as a combination of a plurality of substances which may act individually or together. The term comprises low molecular weight substances and macromolecules, e.g. biopolymers such as nucleic acids and proteins.

Thus, in a further preferred embodiment the method of the invention is suitable for determining the effect of nucleic acids such as genes, transcripts and anti-sense molecules or of proteins on autophagocytosis in order to identify novel target nucleic acids or target proteins for the development of therapeutical agents. In order to achieve this goal, components of the test system, e.g. the source of eukaryotic lysosomes and/or the cytosol extract may be obtained from genetically modified cells, i.e. cells wherein the expression of a gene has been modulated, e.g. by inactivation, mutation, insertion or activation, before the lysosomes or cytosolic extracts have been obtained for the autophagocytosis assay. By means of comparing components from modified cells and from unmodified cells the effect of candidate nucleic acids or candidate proteins on the autophagocystosis may be determined.

Further, the method of the invention comprises the addition of substances, e.g. antibodies, which antagonistically act on components, e.g. proteins in the test system in order to determine the effect of these antagonists or the effect of combinations of antagonists and other test substances as described above on the autophagocytosis.

Further, the invention relates to a test kit for determining, if a test substance is a modulator of autophagocytosis, comprising a cell-free autophagocytosis test system and a dectectable substrate which is capable of being subjected to autophagocytosis in said test system under test conditions.

Another subject matter of the invention is a method for determining autophagocytosis activity in a sample comprising the steps:
a) contacting a cell-free sample with a test reagent containing autophagocytosis-complementing components and a detectable substrate which is capable of being subjected to autophagocytosis under test conditions and
b) determining the autophagocytosis activity in said sample.

This embodiment of the invention relates to a diagnostic method for determining autophagocytosis activity in a sample. This diagnostic method is suitable to determine interferences which are associated with autophagocytosis, e.g. to diagnose starvation and stress conditions, to monitor development and differentiation processes as well as to diagnose diseases associated with cell proliferation such as cell death or tumor diseases, in particular breast cancer.

The diagnostic method of the invention comprises providing a cell-free sample from a biological organism, tissue or body fluid, whose ability of autophagocytosis is to be tested. This sample may contain a source of eukaryotic lysosomes, e.g. a lysosomal preparation containing lysosomes which are capable of autophagocytosis, and/or a source of a cytosolic extract which contains components required for autophagocytosis. Preferably, the cell-free sample is derived from animal tissues and body fluids, particularly from a human. The autophagocytosis activity to be determined may result from the lysosomes and/or the cytosolic extract in the sample.

This sample is contacted with a test reagent containing autophagocytosis-complementing components and a detectable substrate which is capable of being subjected to autophagocytosis under test conditions. The autophagocytosis-complementing components comprise preparations or materials which are required to form an autophagocytosis-competent test system together with the sample to be tested. These components preferably comprise an energy regenerating system, particularly an ATP regenerating system and optionally a cytosol extract and/or a lysosomal preparation from a eukaryotic cell, which may be from the same type of cell from which the lysosomal preparation is obtained or from a different cell, particulary a yeast cell which has been cultured under starvation conditions.

Finally, the present invention comprises a test kit for determining autophagocytosis activity in a sample comprising a test reagent containing autophagocytosis-complementing components and a detectable substrate which is capable of being subjected to autophagocytosis under test conditions.

The invention shall be further explained by the following figures and examples:

### Fig. 1:

Uptake of dextrane coupled FITC by isolated vacuoles. **A)** Isolated vacuoles were incubated in the absence or presence of apyrase (20 U/ml) with 1 *µ*M purified FITC-dextrane (molecular weight approx. 500 kD) under standard reaction conditions. After the indicated periods of time at 27°C, the vacuoles were reisolated, washed, stained with FM4-64 and analyzed by confocal fluorescence microscopy as described in the methods section. The FITC and rhodamine channels of the microscope were superimposed. **B)** Microscopic quantitation of in vitro uptake. A standard uptake reaction with FITC-dextrane was performed as in A, i.e. in the absence or presence of ATP. After 60 min, the vacuoles were reisolated, washed and analyzed by fluorescence microscopy. The graph presents the proportion of vacuoles bearing at least one fluorescent inclusion. **C)** Frequency of autophagic tubes during the reaction. An uptake reaction was performed as in A, i.e. in the presence or absence of ATP. At the indicated times, the vacuoles were reisolated, washed and analyzed by fluorescence microscopy. The frequency of vacuoles bearing a membrane invagination was determined. For B and C five independent experiments were averaged. At least 200 vacuoles from 10 randomly selected fields were analyzed for each data point. Error bars indicate the standard deviation.

### Fig. 2:

Luciferase sequestration as an assay for in vitro uptake. **A)** Luciferase cofractionating with vacuoles is proteinase K protected. In vitro uptake reactions of 3 x the standard volume were performed (60 min, 27°C) in the presence or absence of the ATP regenerating system. The vacuoles were reisolated, washed, resuspended in 150 mM KCI in PS buffer to a concentration of 0.23 mg/ml and split into aliquots. After addition of 1% Triton X-100 or buffer, the mixture was digested with the indicated concentrations of proteinase K (15 min, 0°C). Proteinase treatment was stopped by adding one volume of 0.5 mM PMSF in 150 mM KCI in PS buffer. Then, luciferase activity was assayed. **B)** Luciferase can be released from the vacuolar lumen by freeze-thaw treatment. An uptake reaction was performed (60 min, 27°C). The vacuoles were reisolated and washed as described in the methods section. The final pellet was resuspended in 90 *µ*l PS buffer with 150 mM KCI and split into two aliquots. One was frozen at -80°C and thawed again slowly, whereas the other one remained on ice. The samples were centrifuged (15 min, 145.000 x g, 4°C). The supernatants were recovered and the pellets were resuspended in an equal volume of PS with 150 mM KCI. Luciferase activity in both fractions was determined. The reaction tubes for this experiment had been coated with BSA (0.5 mg/ml, 15 min at room temperature) to reduce unspecific binding of luciferase to the surface.

### Fig. 3:

Time course and temperature dependence of uptake. **A)** Standard uptake reactions were performed in the presence or absence of the ATP regenerating system. After the indicated times at 27°C, the vacuoles were reisolated, washed and protease treated and luciferase which had been taken up was assayed. **B)** Standard uptake reactions were performed for 60 min at the indicated temperature. Luciferase uptake was determined as in A).

### Fig. 4:

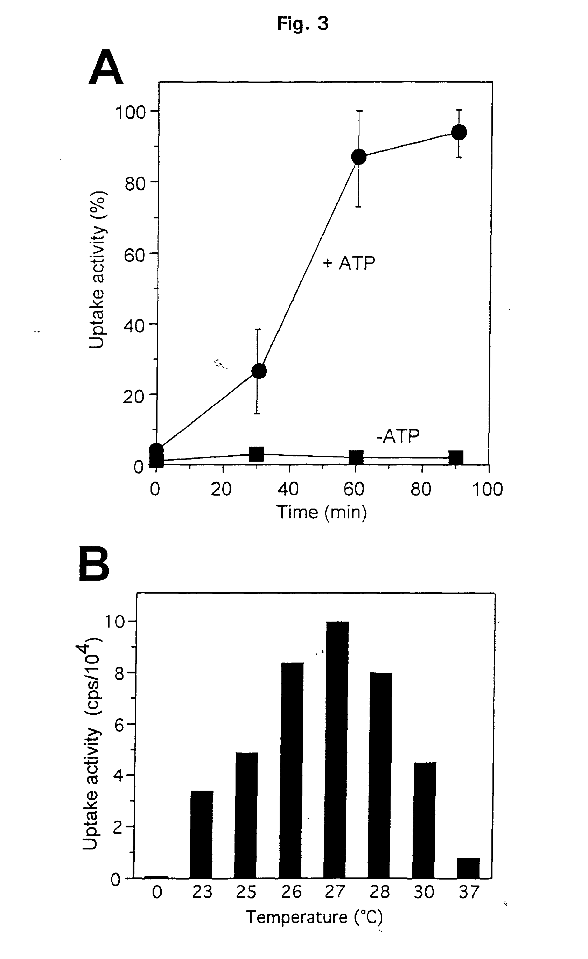

Requirements for in vitro uptake. Standard uptake reactions were performed (60 min, 27°C) in the presence of different concentrations of **A**) cytosol **B**) ATP or **C**) MgCl₂. Luciferase uptake was determined as in Fig. 3A. In A) titration curves for four independent cytosol preparations are shown. The maximal uptake signal of each titration curve was set to 100%. The sample drawn as 0 mM MgCl₂ in C) contained 10 mM EDTA to chelate free magnesium in the reaction.

### Fig. 5:

Influence of cytosols from mutants defective in autophagocytosis. Standard uptake assays were performed (60 min, 27°C) with 3 mg/ml of cytosols derived from wild type (K91-1A) cells, or from isogenic strains with deletions of the indicated genes, both starved on SD(-N) medium to induce autophagocytosis. To assay uptake activity under conditions that do not induce autophagocytosis, one reaction was performed with wild type cytosol prepared from cells grown on rich medium (YPD). Four independent experiments were averaged, using the signal obtained with starved wild type cytosol as 100% reference. Error bars indicate the standard deviation.

### Fig. 6:

In vitro uptake is independent of components involved in homotypic vacuolar fusion. Standard uptake reactions were performed (60 min, 27°C) in the presence of the indicated reagents using a mixture of vacuoles from strains DBY5734-16 (Δpep4) and DBY5734-19 (Δpho8). Therefore, homotypic vacuolar fusion and luciferase uptake could be assayed simultaneously from the same sample. Four independent experiments were averaged. Error bars indicate the standard deviation. For averaging uptake and fusion activities of the control sample without inhibitor were set to 100%. Inhibitors were used at the following concentrations: anti-Sec17p: 1 *µ*M, anti-Sec18p: 1 *µ*M, Gdi1p: 2.5 *µ*M; anti-Vam3p: 1 *µ*M, anti-Vam7p: 1 *µ*M, anti-Nyv1p: 1 *µ*M.

### Fig. 7:

In vitro uptake depends on GTP hydrolysis. Standard uptake reactions were performed in the presence of different concentrations of GTPyS. Luciferase uptake was assayed as in Fig. 3A. Four independent experiments were averaged, using the sample without inhibitor as 100% reference. Error bars indicate the standard deviation. GTPyS did not influence luciferase activity by itself (not shown).

### Examples

### 1. Materials and methods:

### 1.1 Yeast strains and genetic manipulations

DBY5734 *MATa ade2 lys2 his3 trp1 leu2 ura3 cmd1-Δ1::TRP ade3::HIS3::CMD1* (David Botstein); DBY5734-16 *MATa pep4Δ::LEU2 ade2 lys2 his3 trp1 leu2 ura3 cmd1-Δ1::TRP ade3::HIS3::CMD1* DBY5734-19 *MATa pho8Δ::URA3 ade2 lys2 his3 trp1 leu2 ura3 cmd1-Δ1::TRP ade3::HIS3::CMD1.* The following strains are derivatives of DBY5734 obtained by replacement of the indicated genes with a kanamycin resistance cassette: YTS1 *(aut1Δ),* YTS2 *(aut2Δ),* YTS3 *(aut7Δ),* YTS4 *(apg1Δ),* YTS5 *(apg5Δ),* YTS6 *(apg13Δ)*. The strains used for cytosol preparation were derived from K91-1A (Mata pho8::pAL134 pho13::pPH13 lys1; from Y. Kaneko) by replacing the indicated gene with a kanamycin resistance cassette: YTS11 *(aut1Δ),* YTS12 *(aut2Δ)*, YTS13 *(aut7Δ)*, YTS14 *(apg1Δ)*, YTS15 *(apg5Δ)*, YTS16 *(apg13Δ).*

*PHO8* was deleted by one step gene replacement using plasmid pAR2 (J. Shaw, Salt Lake City, Utah) and *PEP4* using plasmid pTS17 (T.Stevens, Eugene, Oregon).

Deletions of AUT and APG genes were obtained as follows: Using the oligonucleotides listed below, DNA fragments for the chromosomal replacement of the corresponding *AUT* or *APG* genes with a *LoxP-KAN*^{*R*}*-LoxP* cassette were created by PCR on plasmid pUG6 (Güldener et al., 1996). The strains YTS1 to YTS6 and YTS11 to YTS16 were made by transforming the amplified single *LoxP-KAN*^{*R*}*-LoxP* cassettes into DBY5734 and K91-1A. Oligonucleotides: aut1ΔKAN1: 5'-CGT ATA TCA AGC TAG CTA GAA GTT AGG AAC AAA GAA GTA CCA GCT GAA AGC TTC GTA CGC -3'; aut1 ΔKAN2: 5'-GTT TAT CCT GTT TTT TGA CCA CCT GGC TTG CAG CTA ATA GGC ATA GGC CAC TAG TGG ATC TG -3'; aut2ΔKAN1: 5'-ATG GAC GAC TTC TTA TCA CGT ATA GGA GTG ATA TAC ATG CAG AGG CAG CTG AAG CTT CGT ACG C-3'; aut2ΔKAN2: 5'-GCA TTT TTC ATC AAT AGG ACT GTG AAT ACC TAC CGT TTC CTT CTC GCA TAG GCC ACT AGT GGA TCT G-3'; aut7ΔKAN1: 5'-CCT GCC AAA TGT ATT TTC TCC TGA GTA AGT GAC ATA CAA AAA CCC CAG CTG AAG CTT CGT ACG C-3'; aut7ΔKAN2: 5'-ATG AAG TCT ACA TTT AAG TCT GAA TAT CCA TTT GAA AAA AGG AAG GCA TAG GCC ACT AGT GGA TCT G-3'; apg1ΔKAN1: 5'-CAT ATT TTC AAA TCT CTT TTA CAA CAC CAG ACG AGA AAT TCA GCT GAA AGC TTC GTA CGC -3'; apg1ΔKAN2: 5'-GGT CAT TTG TAC TTA ATA AGA AAA CCA TAT TAT GCA TCA CGC ATA GGC CAC TAG TGG ATC TG -3'; apg13ΔKAN1: 5'-AAG AAA GCA GAA CAT ACA GCC CGG TTG AAT AGC ATG AGT CCA GCT GAA AGC TTC GTA CGC -3'; apg13ΔKAN2: 5'-TAT TTT TCT TTA GTT GTG CCC TTT AAA ATA AAA CTT TAC CGC ATA GGC CAC TAG TGG ATC TG -3'.

### 1.2 Growth of cells

Yeast was precultured in YPD (1% yeast extract, 2% Bacto-Pepton, 2% glucose) for 6-8 hr at 30°C and then diluted for logarithmic overnight growth (14-16 hr, 30°C, 225 rpm) in 2 l Erlenmeyer flasks with 1 l of YPD medium. For starving cells overnight cultures were harvested at OD₆₀₀ = 2, centrifuged (4 min, 4°C, 3800 x g, JLA 10.500 rotor), washed with sterile water, resuspended in 1 l of SD(-N) medium (0.67% Difco yeast nitrogen base without amino acids and without ammonium sulfate, 2% glucose) and incubated (up to 3 h, 30°C, 225 rpm).

### 1.3 Cytosol preparation

Cytosol was prepared from strain K91-1A or its derivatives. Overnight cultures were harvested at OD₆₀₀ =4.5, centrifuged (4 min, 3800xg, 4°C, JLA 10.500 rotor), washed with sterile water, harvested again, resuspended in 1 I of SD(-N) and incubated for 3 hr at 30°C and 225 rpm. For preparation of cytosol from non-starved cells SD(-N) was replaced by YPD in this incubation. Cells were harvested and washed as above, first with water and then with one pellet volume of chilled lysis buffer (40 mM PIPES-KOH pH 6.8, 0.5 mM MgCl₂, 150 mM KCI, 200 mM sorbitol, 1mM DTT, 0.2 mM PMSF, 0.1 mM pefabloc SC, 0.5 *µ*g/ml pepstatin A, 0.1 *µ*g/ml leupeptin, 50 *µ*M o-phenantroline). After centrifugation (5 min, 3800 x g, 4°C, JLA 10.500 rotor), the pellet was resuspended in a small volume of lysis buffer so that a thick slurry resulted. The suspension was frozen as little nuggets in liquid nitrogen and blended (6-8 times for 30 sec) in a Waring blender filled with liquid nitrogen. Cells were thawed and the lysate was centrifuged (10 min, 12000 x g, 4°C, JA 25.50 rotor).

The supernatant was centrifuged (20 min, 125000 x g, 2°C, TLA45 rotor), the fatty top fraction was discarded and the clarified cytosol was recovered. The protein concentration of this cytosolic fraction was adjusted to 25-30 mg/ml with lysis buffer. Aliquots were frozen in liquid nitrogen and stored at -80°C.

### 1.4 Vacuole preparation

Cells were grown in YPD overnight to an OD₆₀₀ = 2.5-3.0, harvested (4 min, 3800 x g, 4°C, JLA 10.500 rotor) and resuspended in 50 ml of 30 mM Tris/Cl pH 8.9 with 10 mM DTT. Cells were incubated (5 min, 30°C), centrifuged as above, resuspended in 15 ml of spheroplasting buffer (50 mM K-phosphate pH 7.5, 600 mM sorbitol in YPD with 0.2% glucose and oxalyticase [3600 U/ml for DBY5734-16, 2400 U/ml for DBY5734-19 and 3000 U/ml for DBY5734]) and transferred into 30 ml Corex tubes. Cells were incubated (23 min, 30°C), reisolated (1 min, 750 x g and 1 min, 1500xg at 4°C; JA 25.50 rotor) and resuspended in 2.5 ml 15% Ficoll 400 in PS buffer (10 mM PIPES-KOH pH 6.8, 200 mM sorbitol) by gentle stirring with a glass rod. DEAE-dextrane (150 *µ*l for DBY5734-16, 120 *µ*l for DBY5734-19 and 150 *µ*l for DBY5734) was added from a 0.4 mg/ml stock in 15% Ficoll 400 in PS. The spheroplasts were incubated (2 min, 0°C, and 80 sec, 30°C), chilled again, transferred to a SW41 tube and overlaid with 3 ml 8% Ficoll 400, 3 ml 4% Ficoll 400 and 1.5-2 ml 0% Ficoll 400 in PS buffer. After centrifugation (90 min, 154000 x g, 2°C, SW41 rotor), vacuoles were harvested from the 0%/4% interphase.

### 1.5 In vitro autophagocytosis assay

A standard reaction had a volume of 180 *µ*l and was composed of: Vacuoles (0.2 mg/ml, from strain DBY5734), 105 mM KCI, 7 mM MgCl₂, 2 mM ATP, 100 *µ* M DTT, 80 mM Na₂-creatine phosphate, 140 U/ml creatine kinase, 1 x proteinase inhibitor cocktail (PIC; 75 *µ*M pefabloc SC, 75 ng/ml leupeptin, 37.5 *µ*M o-phenanthroline, 375 ng/ml pepstatin A), 17 *µ*g/ml firefly luciferase, 3 mg/ml cytosol from starved cells, 200 mM sorbitol, 10 mM PIPES/KOH pH 6.8. This mixture was incubated for 1 h at 27°C. A 70 *µ*l sample was chilled on ice, diluted with 300 *µ*l 150 mM KCI in PS buffer, centrifuged (6800 x g, 4 min, 2°C, fixed angle table top centrifuge), washed twice with 300 *µ*l 150 mM KCI in PS buffer, and resuspended in 60 *µ*l 150 mM KCI in PS buffer. 10 *µ*l were used to check for complete vacuole recovery by determining the protein concentration or, alternatively, the activity of endogenous Pho8p (according to Haas et al., 1994). 40 *µ*l of the suspension were treated with proteinase K (15 min, 0°C, 275 *µ*g/ml, added from a 5 mg/ml stock in PS) to digest surface-bound luciferase. Digestion was terminated by adding an equal volume of freshly prepared PMSF solution (0.5 mM PMSF, 150 mM KCI, in PS buffer). Luciferase activity was determined from a 25 *µ*l aliquot in a luminometer using a luciferase kit (Berthold Detection Systems, Pforzheim) according to the instructions of the supplier.

### 1.6 In vitro fusion

When fusion was to be determined in addition to autophagocytosis, the standard assay was performed as above, but a 1:1 mixture of vacuoles from a Δpep4 and a Δpho8 strain were used. The former carry pro-alkaline phosphatase (pro-Pho8p), and the latter only the appropriate maturation enzyme (Pep4p). Alkaline phosphatase acitvity is only generated upon mixing of the contents of both vacuole types by fusion (Haas et al., 1994). Fusion was quantitated by taking a 30 *µ*l aliquot out of the standard assay (after incubation for 1 h at 27°C) and determining alkaline phosphatase activity as published (Haas et al., 1994).

### 1.7 Purification of fluorescein dextrane 500000

10 mg fluorescein dextrane 500000 (Molecular Probes, Leiden) were dissolved in 1 ml of PS buffer and concentrated and rediluted at least five times by ultrafiltration in Centricon 30 devices. The retentate was used for the experiments if low molecular weight fluorescein was no longer detectable in the filtrate by fluorescence spectroscopy in a Perkin Elmer LS 50-B spectrometer.

### 1.8 Microscopic assay of autophagocytosis in vitro

The standard assay was modified by using 1 *µ*M purified fluorescein dextrane 500000 (20 *µ*M stock) instead of luciferase. Samples were taken, and the vacuoles were reisolated and washed as described for the chemical assay. The vacuoles were resuspended in PS buffer with 50 *µ*M FM4-64 to a protein concentration of approximately 1 mg/ml. The suspension was mixed with an equal volume of 0.4% Seaplaque agarose in PS (kept liquid at 35°C). 12 *µ* l were transferred to a slide, chilled at 4°C for 5 min to immobilize the vacuoles and analyzed by confocal fluorescence microscopy on a Leica TCS system, or by conventional fluorescence microscopy.

### 2. Results

### 2.1 Reconstitution of vacuolar invagination and solute uptake in vitro

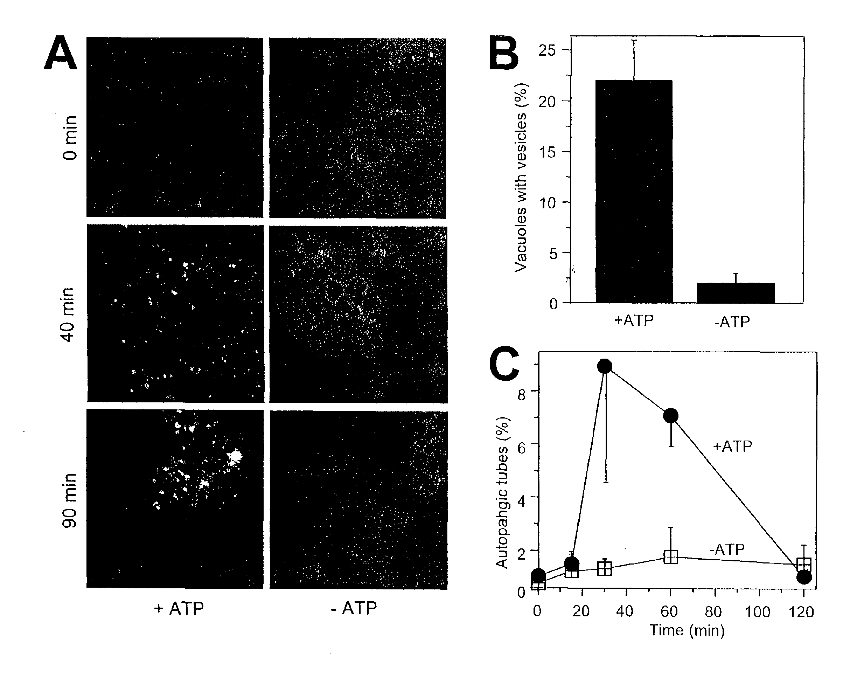

Microautophagocytosis occurs by direct invagination of lysosomal membranes, an "inverted" budding process into the lumen of the organelle. Soluble material is entrapped inside the invaginating vesicle and - upon scission of the membrane - becomes inaccessible to the surrounding medium. We have reconstituted this process in vitro using purified vacuoles from yeast. These organelles can be isolated in very good purity and yield (Wiemken, 1975) and can readily be observed by light microscopy. We incubated isolated vacuoles in the presence of a cytosolic extract, an ATP regenerating system, and appropriate salt and buffer conditions. In order to monitor vesicular uptake of soluble material from the medium, the incubation was performed in the presence of high molecular weight dextrane coupled to FITC. Dye which had not been taken up was removed by reisolating the vacuoles and uptake was analyzed by fluorescence microscopy. After 60 min of incubation almost a quarter of the vacuoles contained punctate structures (Fig. 1A, B) which were mobile in the lumen. Formation of the FITC-dextrane containing vesicles was ATP-dependent. Upon longer incubation these vesicles disappeared. The vacuolar lumen became homogeneously stained (Fig. 1A), indicating that the vesicles had been lysed inside the vacuoles and their contents dispersed in the vacuolar lumen. Vacuoles were also stained with the membrane dye FM4-64 to assay for the presence of autophagic tubes, the tubular membrane invaginations from which vesicles bud into the lumen of vacuoles in vivo (see accompanying manuscript). At the beginning of the incubation, less than 1 % of the vacuoles had autophagic tubes (Fig. 1C). After a lag-phase of 15 min, the frequency of autophagic tubes increased to almost 10%. Beyond 60 min of incubation, the tubes disappeared gradually. The formation of autophagic tubes was ATP dependent, indicating that it was an active process. We conclude that the in vitro reaction reconstitutes the formation of autophagic tubes and the vesicular uptake of soluble material into vacuoles.

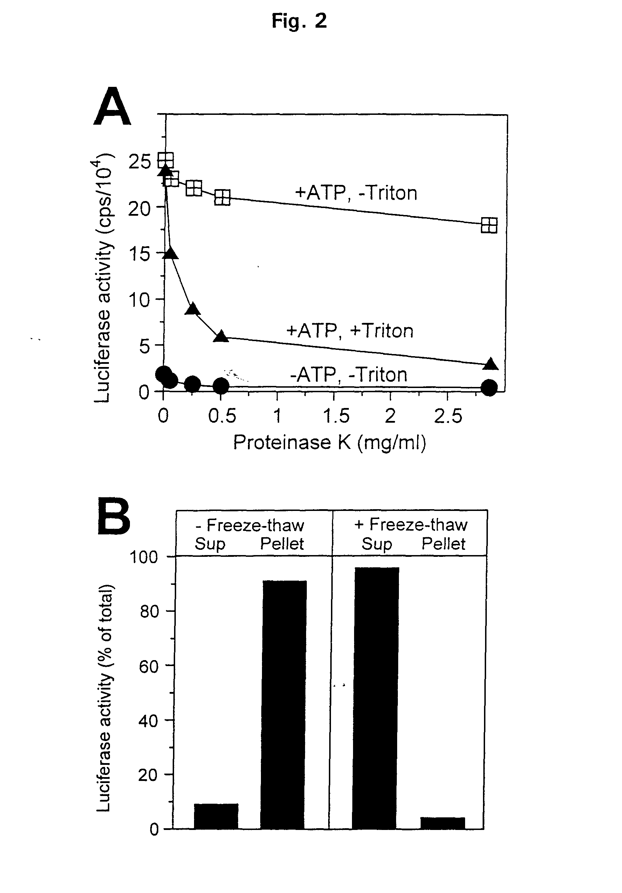

Using these microscopical assays, the concentrations of all components used had been optimized to yield maximal uptake activity (data not shown). The results of these optimizations were identical to those obtained with the chemical uptake assay described below. We developed this assay, which is based on the uptake of firefly luciferase, to provide a means for more convenient and sensitive quantitation of vesicle formation. Isolated vacuoles were incubated with cytosol and ATP in the presence of purified firefly luciferase. The organelles were reisolated, washed and treated with increasing concentrations of proteinase K to digest residual luciferase potentially adhering to the surface of the vacuoles. Only luciferase which had been taken up into the vacuoles would resist this treatment. A strong signal of proteinase K protected luciferase was recovered from samples incubated in the presence of ATP (Fig. 2A). In the absence of ATP, no protease protected luciferase cofractionated with the vacuoles. The protease protected luciferase became accessible to proteinase K after lysis of the vacuoles by detergent (Fig. 2A), or after rupturing the membranes in a freeze-thaw step (Fig. 2B). This demonstrates that protease protection was due to sequestration into the vacuolar lumen. Therefore, the behaviour of luciferase matched that of FITC-dextrane and could be used as a measure for membrane invagination and solute uptake from the medium.

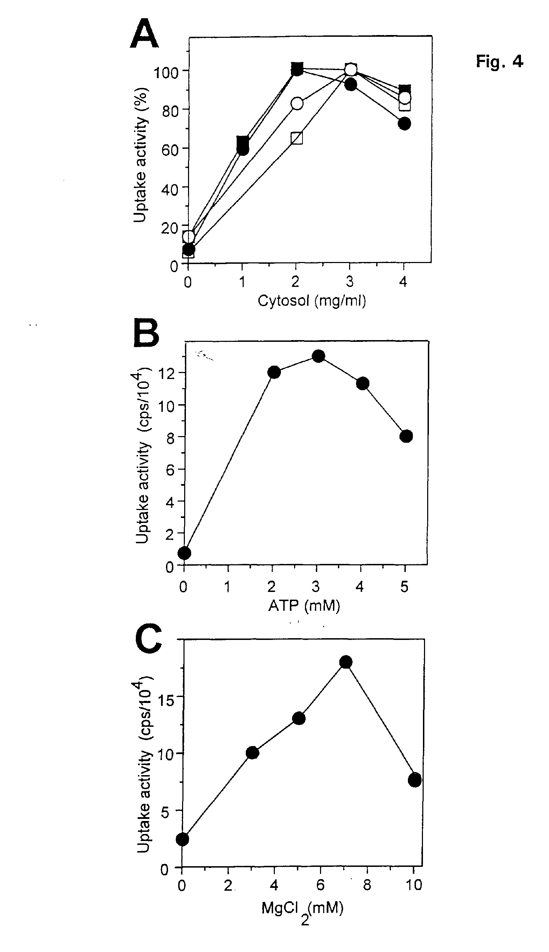

The uptake reaction proceeded for approximately 60 min, with a lag in the first 30 min, and then entered a plateau at 90 min (Fig. 3A). This correlates with the time course of formation and disappearance of autophagic tubes (Fig. 1C). Longer incubation led to a significant reduction of the luciferase signal (not shown), consistent with the fact that the invaginated vesicles became lysed inside the vacuoles (compare Fig. 1A). Luciferase released into the vacuolar lumen can then be degraded by vacuolar proteases. Luciferase uptake was temperature dependent with a narrow optimum at 27°C (Fig. 3B). The reaction had a strong requirement for cytosol, with optima ranging from 2 to 4 mg/ml, depending on the individual batch of cytosol (Fig. 4A). The reaction also required ATP and Mg²⁺, with optima at 2 mM and 7 mM, respectively (Figs 4B, 4C). These optima matched those found independently by the visual assay for vesicular uptake of FITC-dextrane (data not shown), confirming that both assays measure the same process.

### 2.2 Vesicle formation depends on starvation and on gene products required for autophagocytosis

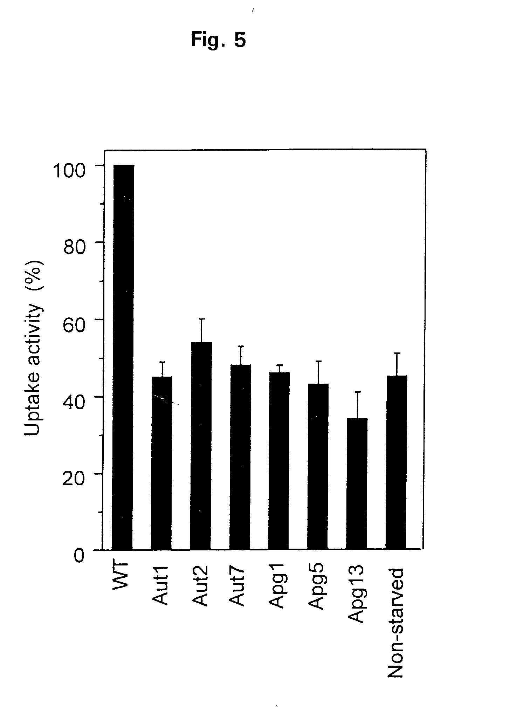

Autophagocytosis in Saccharomyces cerevisiae is a constitutive process which is induced under conditions of stress or starvation (Takeshige et al., 1992; Noda et al., 1995). A large number of mutations influencing this process have been isolated (Thumm et al., 1994; Tsukada and Ohsumi, 1993; Harding et al., 1995). Since none of the genes identified so far codes for a protein predicted to be an integral membrane component, we tested the activity of cytosols from mutant strains in the in vitro reaction. Deletion mutants for AUT1, AUT2, AUT7/APG8, APG1, APG5 and APG13 and the wild-type control were starved for nitrogen on SD(-N) medium. Cytosol prepared from any of the mutant strains had a significantly lower activity in the in vitro assay than wild-type cytosol (Fig. 5). Also the activity of cytosol from wild-type cells grown on a rich nitrogen source, i.e. under conditions not inducing autophagocytosis, was 50-60% lower than that of cytosol from starved wild-type cells (Fig. 5). Performing the assays with vacuoles isolated from Apg/Aut mutants, or from starved wild type cells, did not increase the difference (not shown). Starvation and the Apg/Aut pathway do obviously not produce persistent changes on the vacuolar membrane. Their regulatory influences can be reset by the cytosol during the incubation. In summary, isolated vacuoles perform the uptake of macromolecular solutes into vesicular structures inside the organelle. The system reproduces the induction of autophagocytosis by starvation and the influence of the Apg/Aut proteins. Therefore, the in vitro reaction reconstitutes microautophagocytosis.

### 2.3 Vesicle formation is independent of proteins mediating homotypic vacuolar fusion, biosynthetic transport to the vacuole, and macroautophagocytosis

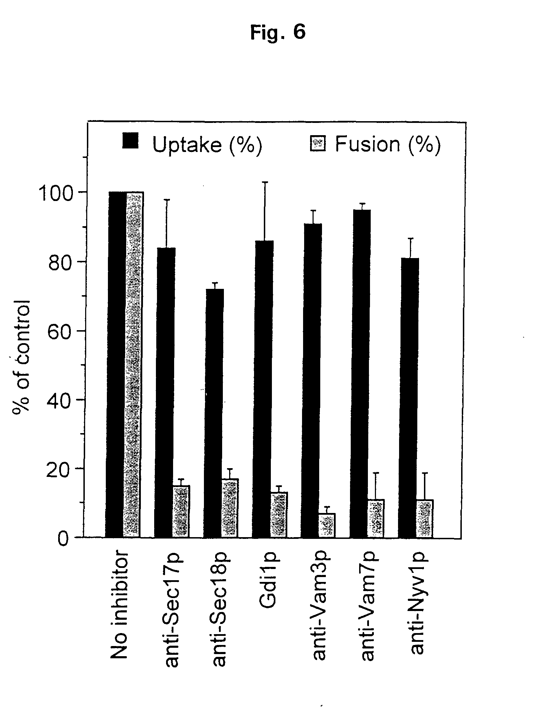

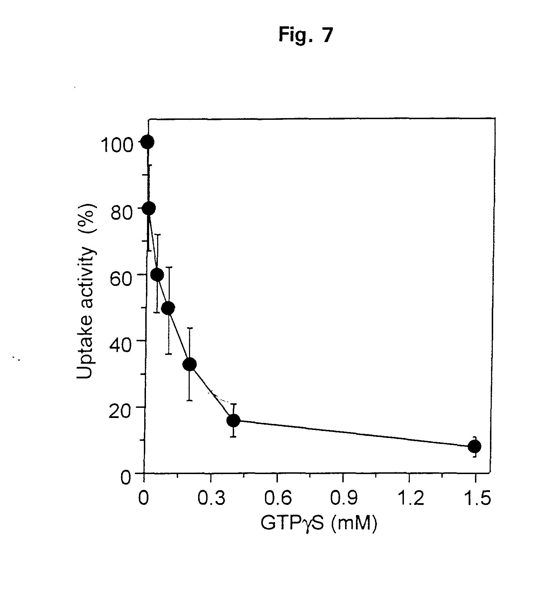

Scission of the invaginating membrane to form a vesicle is functionally a homotypic fusion reaction of the vacuolar membrane in the autophagic tube. Homotypic vacuolar fusion is also part of the normal physiology of the vacuolar membrane. During transmission into the growing daughter cell, the vacuole undergoes a regulated cycle of fragmentation into smaller vesicles, transport of these structures into the bud, and their homotypic fusion to reconstitute the new vacuole (Warren and Wickner, 1996). This homotypic fusion event has been characterized in vitro and in vivo and many components involved have been identified. They include, among others, the SNAREs Vam3p, Vam7p and Nyv1p, Sec17p (α-SNAP), Sec18p (NSF) and Ypt7p (a Rab protein) (Nichols et al., 1997; Ungermann and Wickner, 1997; Haas et al., 1996 and 1995). Vam3p and Vam7p are also involved in biosynthetic transport to the vacuole and in macroautophagocytosis (Wada et al., 1992; Sato et al., 1998; Darsow et al., 1997; Srivastava and Jones, 1998). We asked whether these components would be required for the homotypic fusion process necessary to pinch off the invaginated membrane and release vesicles into the vacuolar lumen. An in vitro uptake reaction was performed in the presence of affinity purified antibodies to Sec17p, Sec18p, Vam3p, Vam7p or Nyv1p, or in the presence of Gdi1p which extracts Ypt7p from the membrane. One aliquot of the reaction was used to quantify the uptake of luciferase into the vacuoles. A second aliquot of the same sample was used to monitor homotypic vacuolar fusion and control for the effect of the inhibitors. Vacuole fusion occurs under the conditions of the uptake reaction, albeit at only 20-30% of the efficiency obtainable under optimized conditions (Mayer et al., 1996). None of the inhibitors had a strong influence on the uptake of luciferase into the vacuolar lumen (Fig. 6) although they blocked the homotypic fusion of vacuoles thoroughly. Like vacuole fusion (Haas et al., 1994), however, luciferase uptake depended on guanine-nucleotides. Vesicle formation was prevented by the poorly hydrolysable GTP analogue GTPyS with an IC₅₀ of 100 *µ*M (Fig. 7), indicating that GTP hydrolysis is required.

We conclude that the invagination and scission of vesicles into the vacuolar lumen involves the action of a GTPase. It occurs independently of homotypic vacuolar fusion, of biosynthetic transport to the vacuole, and of macroautophagocytosis, but it is subjected to control via starvation and components of the Apg/Aut pathway.

### 3. Discussion

Transport of proteins into yeast vacuoles can occur via multiple pathways: vesicular transport from endosomes along the alkaline phosphatase, carboxypeptidase Y, or multivesicular body pathways (Wendland et al., 1998); the Cvt pathway, defined by aminopeptidase I (Scott and Klionsky, 1998); the Vid pathway leading to degradation of fructose-1,6-bisphosphatase by inclusion into a novel type of vesicles (Huang and Chiang, 1997); direct Hsp70p-dependent translocation across the vacuolar membrane (Horst et al., 1999); and macroautophagocytosis (Takeshige et al., 1992). We have recently identified a microautophagic pathway in Saccharomyces cerevisiae which operates via a specialized structure, the autophagic tube (see accompanying manuscript). This tube is a narrow and long invagination which is filled with cytosol and pinches off vesicles into the lumen of the vacuole. The tip of the tube, where vesicles pinch off, is free of transmembrane particles. The smooth ultrastructural appearance of this zone of vesiculation matches that of autophagic bodies which are also devoid of transmembrane particles (compare accompanying manuscript and Baba et al., 1995).

The relative contributions of the micro- and macroautophagic pathway to total protein transfer into the vacuole are not known and are hard to estimate. We found that the microautophagic uptake of solutes into isolated vacuoles is independent of the t-SNAREs Vam3p and Vam7p which are required for macroautophagocytosis, for the Cvt, alkaline phosphatase, carboxypeptidase Y and multivesicular body pathway (Darsow et al., 1997; Srivastava et al., 1998; Sato et al., 1998; Abeliovich, 1999). Cells temperature sensitive for Vam3p function and deficient for the protease Pep4p accumulate autophagosomes in the cytosol within three hours of starvation at the non-permissive temperature. They do not, however, accumulate autophagic bodies in the lumen of the vacuole. Therefore, the rate of microautophagic uptake into vacuoles must be lower than the rate of degradation of autophagic bodies inside the vacuoles of PEP4 mutants. The proteolytic activity of such mutant vacuoles is reduced, but not absent (Jones et al., 1982; Teichert et al., 1989). The inability of VAM3 ts mutants to accumulate autophagic bodies also suggests that the rate of fluid phase uptake via microautophagocytosis is considerably lower than that of macroautophagocytosis. Alternatively, autophagic bodies resulting from microautophagocytosis might be degraded more rapidly than those from macroautophagocytosis. Furthermore, full induction of microautophagocytosis may require longer starvation. Longer time points cannot be assayed with VAM3 ts mutants, because the vacuoles in these cells desintegrate upon incubation at non-permissive temperature for more than three hours (Darsow et al., 1997). Rigorous determination of the fluxes through the macro- and microautophagic pathways will require the identification of selective inhibitors for both routes.

Ultrastructural analysis revealed that the nascent microautophagic vesicles have a strikingly low density of transmembrane particles (see accompanying manuscript). This unusual structure correlates with the unexpected biochemical properties of vesicle formation. Microautophagic vesicle scission into the lumen of the vacuole is functionally a homotypic membrane fusion event. Despite this, it is independent of components catalyzing homotypic fusion between vacuoles, such as the SNAREs Vam3p, Vam7p and Nyv1 p. Independence of Vam3p distinguishes scission also from heterotypic fusion events occurring between vacuoles and vesicular intermediates of e.g. Pho8p or CPY transport, or of macroautophagocytosis (Darsow et al., 1997). Microautophagic vesicle scission is even independent of Sec18p (NSF) and Sec17p (α-SNAP) which are involved in most intracellular fusion events. Therefore, this reaction does probably not depend on the highly conserved apparatus for intracellular membrane fusion at all.

An.attractive speculation is that scission may be lipid-driven. Scission of synaptic vesicles undergoing endocytosis requires the concerted action of the GTPase dynamin and endophilin, a lysophosphatidic acid acyl transferase (Schmidt et al., 1999). The action of this enzyme converts an inverted-cone-shaped lipid (lysophosphatidic acid) into a cone-shaped lipid (phosphatidic acid) in the cytoplasmic leaflet of the bilayer, a process which could support formation as well as scission of the nascent vesicle. It is unlikely that the same mechanism acts also on microautophagic vesicles because microautophagic scission is topologically inverse. In endocytosis enzymes involved in vesicle formation and pinching bind to the outer side of the vesicle and its neck and induce negative curvature of the cyoplasmic leaflet. In microautophagocytosis, however, they would have to bind to the inside of the nascent structure and locally increase the curvature of the cytoplasmic leaflet. Topologically, the microautophagic vesicle formation is more related to the formation of multivesicular bodies, i.e. the formation of internal endosomal vesicles. Those vesicles are strongly enriched in a specialized lipid, lysobisphosphatidic acid (Kobayashi et al., 1998). The smooth appearance of the fractured membrane of both vacuolar autophagic bodies and the zone of vesicle formation on autophagic tubes suggests that both membranes are poor in transmembrane proteins and therefore lipid rich. Since vesicle formation is also independent of the enzymes catalyzing homotypic vacuolar membrane fusion, lipids may well play an important part in the budding of vesicles into the vacuolar lumen. The in-vitro system described here will be an important tool to address this question.

### References

Abeliovich, H., Darsow, T. and Emr, S.D. (1999) Cytoplasm to vacuole trafficking of aminopeptidase I requires a t-SNARE-Sec1p complex composed of Tlg2p and Vps45. EMBO J. 18, 6005-6016.

Baba, M., K. Takeshige, N. Baba and Y. Ohsumi (1994) Ultrastructural analysis of the autophagic process in yeast: Detection of autophagosomes and their characterization. J. Cell Biol. 124,903-913.

Baba, M., Osumi, M. and Ohsumi Y (1995) Analysis of the membrane structures involved in autophagy in yeast by freeze-replica method. Cell Structure & Function. 20,465-71.

Baba, M., Osumi, M., Scott, S.V., Klionsky, D.J. and Ohsumi, Y. (1997) Two distinct pathways for targeting proteins from the cytoplasm to the vacuole/lysosome. J. Cell Biol. 139, 1687-95.

Cuervo, A.M. and Dice, JF. (1998) Lysosomes, a meeting point of proteins, chaperones, and proteases. J. Mol. Med. 76, 6-12

Darsow, T., Rieder, S.K. and Emr, S.D. (1997) A multispecificity syntaxin homologue, Vam3p, essential for autophagic and biosynthetic protein transport to the vacuole. J. Cell Biol. 138, 517-529.

Dunn, W.A. Jr. (1990) Studies on the mechanisms of autophagy: Formation of the autophagic vacuole. J. Cell Biol. 110,1923-1933.

Güldener, U., Heck, S., Fiedler, T., Beinhauer, J. and Hegemann, J.H. (1996) A new efficient gene disruption cassette for repeated use in budding yeast. Nucl. Acid Res. 24, 2519-2524

Haas, A., Conradt, B. and Wickner, W. (1994) G-protein ligands inhibit in vitro reactions of vacuole inheritance. J. Cell Biol., 126, 87-97.

Haas, A., Schleglmann, D., Lazar, T., Gallwitz, D., and Wickner, W. (1995) The GTPase Ypt7 of Saccharomyces cerevisiae is required on both partner vacuoles for the homotypic fusion step of vacuole inheritance. EMBO J. 14, 5258-5270.

Haas, A. and Wickner, W. (1996) Homotypic vacuole fusion requires See17p (yeast α-SNAP) and Sec18p (yeast NSF). EMBO J. 15,3296-3305

Harding, T.M., Morano, K.A., Scott, S.V., and Klionsky, D.J. (1995) Isolation and characterization of yeast mutants in the cytoplasm to vacuole protein targeting pathway. J. Cell Biol. 131,591-602

Harding, T.M., Hefner-Gravink, A., Thumm, M. and Klionsky, D.J. (1996). Genetic and phenotypic overlap between autophagy and the cytoplasm to vacuole protein targeting pathway. J. Biol. Chem. 271, 17621-17624.

Horst, M., Knecht, E.C. and Schu, P.V (1999) Import into and degradation of cytosolic proteins by isolated yeast vacuoles. Mol. Biol. Cell. 10, 2879-2889.

Huang, P.H. and Chiang, H.L. (1997) Identification of novel vesicles in the cytosol to vacuole protein degradation pathway. J. Cell Biol. 136,803-10

Jones, E.W., Zubenko, G.S. and Parker, R.R. (1982) PEP4 gene function is required for expression of several vacuolar hydrolases in Saccharomyces cerevisiae. Genetics. 102, 665-77

Kim, J., Dalton, V., Eggerton, V., Scott, S.V. and Klionsky, D.J. (1999) Apg7p/Cvt2p is required for the cytoplasm-to-vacuole targeting, macroautophagy, and peroxisome degradation pathways. Mol. Biol. Cell 10, 1337-1351.

Kirisako, T., Baba, M., Ishihara, N., Kouichi, M., Ohsumi, M., Yoshimori, T., Noda, T., and Ohsumi, Y. (1999) Formation process of autophagosome is traced with Apg8/Aut7p in yeast. J. Cell Biol. 147, 435-446.

Knop, M., Schiffer, H.H., Rupp, S. and Wolf, D.H. (1993) Vacuolar/lysosomal proteolysis: proteases, substrates, mechanisms. Curr. Op. Cell Biol. 5, 990-996

Kobayashi, T., Stang, E., Fang, K.S., Demoerloose, P., Parton, R.G. and Gruenberg, J. (1998) A lipid associated with the antiphospholipid syndrome regulates endosome structure and function. Nature. 392, 193-197

Lang, T., Schaeffeler, E., Bernreuther, D., Bredschneider, M., Wolf, D.H. and Thumm, M. (1998) Aut2p and Aut7p, two novel microtubule-associated proteins are essential for delivery of autophagic vesicles to the vacuole. EMBO J. 17, 3597-3607.

Lenk, S.E., Dunn, W.A., Trausch, J.S., Ciechanover, A. and Schwartz, A.L. (1992). Ubiquitin-activating enzyme E1 is associated with maturation of autophagic vacuoles. J. Cell Biol. 118, 301-308.

Mayer, A., Wickner, W. and Haas, A. (1996) Sec18p (NSF)-driven release of Sec17p (α-SNAP) precedes docking and fusion of yeast vacuoles. Cell 85, 83-94.

Matsuura, A., Tsukada, M., Wada, Y. and Ohsumi, Y. (1997) Apg1p, a novel protein kinase required for the autophagic process in Saccharomyces cerevisiae. Gene. 192, 245-250.

Mizushima, N., Noda, T., and Ohsumi, Y. (1999) Apg16p is required for the function of the Apg12p-Apg5p conjugate in the yeast autophagy pathway. EMBO J. 18, 3888-96

Mizushima, N., Noda, T., Yoshimori, T., Tanaka, Y., Ishii, T., George, M.D., Klionsky, D.J., Ohsumi, M. and Ohsumi, Y. (1998) A protein conjugation system essential for autophagy Nature 395, 395-8.

Mortimore, G.E. and Kadowaki, M. Autophagy: Its mechanism and regulation. In: Ciechanover, A.J. and Schwartz, A.L. (Eds., 1994) Cellular Proteolytic Systems. Wiley & Liss, New York.

Nichols, B.J., Ungermann, C., Pelham, H.R.B., Wickner, W.T., and Haas A. (1997) Homotypic vacuolar fusion mediated by t- and v-SNAREs. Nature 387, 199-202

Noda, T., Matsuura, A., Wada, Y. and Ohsumi, Y. (1995) Novel system for monitoring autophagy in the yeast Saccharomyces cerevisiae. Biochem. Biophys. Res. Comm. 210, 126-132.

Odorizzi, G., Babst, M., and Emr, S.D. (1998) Fab1p Ptdlns(3)P 5-kinase function essential for protein sorting in the multivesicular body. Cell 95, 847-858.

Rothman, J. E. (1994) Mechanisms of intracellular protein transport. Nature 372, 55-63.

Sakai, Y., Koller, A., Rangell, L.K., Keller, G.A. and Subramani, S. (1998) Peroxisome degradation in Pichia pastoris: Identification of specific steps and morphological intermediates. J. Cell Biol. 141,625-636.

Sato, T.K., Darsow, T. and Emr, S.D. (1998) Vam7p, a SNAP-25-like molecule, and Vam3p, a syntaxin homolog, function together in yeast vacuolar protein trafficking. Mol. Cell. Biol. 18,5308-5319

Schmidt, A., Wolde, M., Thiele, C., Fest, W., Kratzin, H., Podtelejnikov, A.V., Witke, W., Huttner, W.B. and Söhling, H.D. (1999) Endophilin I mediates synaptic vesicle formation by transfer of arachidonate to lysophosphatidic acid. Nature. 401, 133-141.

Scott, S.V., Hefner-Gravink, A., Morano, K, Noda, T., Ohsumi, Y., Klionsky, D.J. (1996). Cytoplasm to vacuole targeting and autophagy employ the same machinery to deliver proteins to the yeast vacuole. Proc. Natl. Acad. Sci. 93, 12304-12308.

Seglen, P.O. and Bohley, P. (1992) Autophagy and other vacuolar protein degradation mechanisms. Experientia 48,158-172.

Shintani, T., Mizushima, N., Ogawa, Y., Matsuura, A., Noda, T. and Ohsumi, Y. (1999) Apg 10p, a novel protein-conjugating enzyme essential for autophagy in yeast. EMBO J. 18, 5234-5241

Srivastava, A. and Jones, E.W. (1998) Pth1/Vam3p is the syntaxin homolog at the vacuolar membrane of Saccharomyces cerevisiae required for the delivery of vacuolar hydrolases. Genetics 148,85-98.

Straub, M., Bredschneider, M., and Thumm, M. (1997) AUT3, as serine/threonine kinase gene, is essential for autophagocytosis in Saccharomyces cerevisiae. J. Bac. 179, 3875-3883.

Stromhaug, P.E., Berg, T.O., Fengsrud, M., and Seglen, P.O. (1998) Purification and characterization of autophagosomes from rat hepatocytes. Biochem. J. 335, 217-224

Takeshige, K., M. Baba, S. Tsuboi, T. Noda and Y. Ohsumi (1992) Autophagy in yeast demonstrated with proteinase-deficient mutants and conditions for its induction. J. Cell Biol. 119,301-311.

Teichert, U., Mechler, B., Müller, H. and Wolf, D.H. (1989) Lysosomal (vacuolar) proteinases of yeast are essential catalysts for protein degradation, differentiation, and cell survival. J. Biol. Chem. 264, 16037-16045.

Thumm, M., R. Egner, B. Koch, M. Schlumpberger, M. Straub, M. Veenhuis and D.H. Wolf (1994). Isolation of autophagocytosis mutants of Saccharomyces cerevisiae. FEBS Lett. 349,275-280.

Titorenko V.I., Keizer, I., Harder, W. and Veenhuis, M. (1995) Isolation and characterization of mutants impaired in the selective degradation of peroxisomes in the yeast Hansenula polymorpha. J. Bac. 177, 357-363.

Tsukada, M. and Y. Ohsumi (1993) Isolation and characterization of autophagy-defective mutants of Saccharomyces cerevisiae. FEBS Lett. 333,168-174.

Tuttle, D.L., A.S. Lewin and W.A. Dunn Jr. (1993) Selective autophagy of peroxisomes in methylotrophic yeasts. Eur. J. Biochem. 60,283-290.

Tuttle, D.L. and Dunn, W.A. Jr. (1995) Divergent modes of autophagy in the methylotrophic yeast Pichia pastoris. J. Cell Sci. 108,25-35.

Ungermann, C. and Wickner, W. (1998) Vam7p, a vacuolar SNAP-25 homolog, is required for SNARE complex integrity and vacuole docking and fusion. EMBO J. 17,3269-3276.

Veenhuis, M., Douma, A., Harder, W. and Osumi, M. (1983) Degradation and turnover of peroxisomes in the yeast Hansenula polymorpha induced by selective inactivation of peroxisomal enzymes. Arch. Microbiol. 134, 193-203.

Wada, Y., Ohsumi, Y., and Anraku, Y. (1992) Genes for directing vacuolar morphogenesis in Saccharomyces cerevisiae. J. Biol. Chem. 267, 18665-18670.

Warren, G. and Wickner, W. (1996) Organelle inheritance. Cell 84, 395-400

Wendland, B., Emr, S.D. and Riezman, H. (1998) Protein traffic in the yeast endocytic and vacuolar protein sorting pathways. Curr. Op. Cell Biol. 10, 513-22.

Wiemken A. (1975) Isolation of vacuoles from yeasts. Meth. Cell Biol. 12, 99-109.

Yamamoto, A., R. Masaki and Y. Tashiro (1990a) Characterization of the isolation membranes and the limiting membranes of autophagosomes in rat hepatocytes by lectin cytochemistry. J. Histochem. Cytochem. 38, 573-580.

Yamamoto, A., R. Masaki and Y. Tashiro (1990b) Absence of cytochrome P-450 and presence of autolysosomal membrane antigens on the isolation membranes and autophagosomal membranes in rat hepatocytes. J. Histochem. Cytochem. 38,1571-1581.

Yokota S. (1993) Formation of autophagosomes during degradation of excess peroxisomes induced by administration of dioctyl phtalate. Eur. J. Cell Biol. 61, 67-80

Yuan, W., Stromhaug, P.E. and Dunn, W.A. (1999) Glucose-induced autophagy of peroxisomes in Pichia pastoris requires a unique E1-like protein. Mol. Biol. Cell 10, 1353-1366.

## Claims

1. A method for determining, if a test substance is a modulator of autophagocytosis, comprising the steps:
a) providing a cell-free autophagocytosis test system and a detectable substrate which is capable of being subjected to autophagocytosis in said test system under test conditions and
b) determining the influence of the test substance on the autophagocytosis of said substrate.

2. The method of claim 1 wherein said cell-free autophagocytosis test system comprises a source of eukaryotic lysosomes, a cytosol extract from a eukaryotic cell and an ATP regenerating system.

3. The method of claim 2 wherein said source of lysosomes comprises vacuoles from a yeast cell.

4. The method of claim 2 wherein said cytosol extract is a cytosol extract from a yeast cell.

5. The method of claim 2 wherein said ATP regenerating system comprises ATP, creatine phosphate and creatine kinase.

6. The method of any one of claims 1-5 wherein said detectable substrate is a fluorescent substrate, a reporter enzyme, a radiolabeled substrate or a coloured substrate.

7. The method of claim 6 wherein said substrate is selected from fluorescence-labeled compounds and reporter enzymes.

8. The method of claim 7 wherein said substrate is selected from fluorescein-labeled dextrane and firefly luciferase.

9. The method of any one of claims 1-8 wherein step b) comprises selectively inactivating non-autophagocytosized substrate and subsequently determining the autophagocytosized substrate.

10. A test kit for determining, if a test substance is a modulator of autphagocytosis, comprising a cell-free autophagocytosistestsystem and a detectable substrate which is capable of being subjected to autophagocytosis in said test system under test conditions.

11. A method for determining autophagocytosis activity in a sample comprising the steps:
a) contacting a cell-free sample with a test reagent containing autophagocytosis-complementing components and a detectable substrate which is capable of being subjected to autophagocytosis under test conditions and
b) determining the autophagocytosis activity in said sample.

12. The method of claim 11 wherein said cell-free sample is derived from animal tissues and body fluids.

13. The method of claim 12 wherein said cell-free sample is derived from a human.

14. The method of any one of claims 11-13 wherein said autophagocytosis-complementing components comprises an ATP regenerating system.

15. A test kit for determining autophagocytosis activity in a sample comprising a test reagent containing autophagocytosis-complementing components and a detectable substrate which is capable of being subjected to autophagocytosis under test conditions.
